# FASCICULE DE BREVET EUROPEEN

(11) **EP 2 747 739 B1**
(45) Date de publication et mention de la délivrance du brevet: **02.09.2015**
(21) Numéro de dépôt: 13700114.5
(22) Date de dépôt: 10.01.2013
(51) Int. Cl.: A61K 8/02, A61K 8/19, A61K 8/49, A61K 8/60, A61Q 19/08

(54) **COMPOSITIONS DERMO-COSMÉTIQUES À BASE D'UNE ASSOCIATION SYNERGIQUE D'ARGENT COLLOÏDAL ET D'ACIDE DÉSOXYRIBONUCLÉIQUE**
DERMOKOSMETISCHE ZUSAMMENSETZUNGEN AUF DER BASIS EINER SYNERGISTISCHEN KOMBINATION AUS KOLLOIDALEM SILBER UND DESOXYRIBONUKLEINSÄURE
DERMOCOSMETIC COMPOSITIONS BASED ON A SYNERGISTIC COMBINATION OF COLLOIDAL SILVER AND DEOXYRIBONUCLEIC ACID

(30) Priorité: 16.01.2012 FR 1200122
(43) Date de publication de la demande: 02.07.2014
(73) Titulaire: Argentum Holding S.a.r.l., 2540 Luxembourg (LU)
(72) Inventeur: MOUZIN, Gilbert, F-81100 Castres (FR); THIERRY, Stéphanie, London NW10 1LB (GB); ISAACS, Joy, London NW6 7DN (GB)
(74) Mandataire: Dennemeyer & Associates S.A.
(86) Numéro de dépôt international: PCT/EP2013/050422
(87) Numéro de publication internationale: WO 2013/107687

(56) Documents cités:
- EP-A2- 1 464 962
- WO-A1-2008/079898
- WO-A2-2004/071538
- JP-A- 2008 063 295

## Description

La présente invention concerne des compositions dermo-cosmétiques selon la revendication 1 et l'utilisation de telles compositions en cosmétologie selon la revendication 12.

La présente invention concerne de nouvelles formulations topiques utilisables en dermo-cosmétologie et plus particulièrement dans le traitement de la sénescence cutanée physiologique et actinique.

Ces formulations sont à base d'une association de principes actifs qui potentialisent la régénération du collagène, ainsi que la protection contre les radicaux libres.

Les principes actifs sont principalement constitués par de l'argent colloïdal et de l'acide désoxyribonucléique (ADN) éventuellement en association avec de la caféine.

WO2008079898 concerne une méthode et une formulation topique comprenant un métal colloïdal pour le traitement ou la prévention de la peau.

EP1464962 décrit des complexes de biomolécules d'argent colloïdal.

JP2008063295 concerne une préparation de soin de la peau contenant du platine/argent colloïdal.

WO2004071538 décrit un procédé de libération d'agents cosmétiques par application topique.

### Le vieillissement de la peau:

### Vieillissement physiologique :

De nombreux phénomènes sont imbriqués : le déterminisme génétique, le mode de vie, l'état général de l'organisme s'ajoutent à l'âge pour altérer les structures physiques et le fonctionnement du revêtement cutané.

La première manifestation qui apparaît vers 30 ans touche les fibres élastiques du derme papillaire. La seconde étape apparaît vers 50 ans concerne les fibres du collagène du derme profond.

### Vieillissement actinique:

Le soleil accentue les rides et fait apparaître des taches pigmentées sur la peau.

L'examen au microscope d'une zone de peau exposée de façon chronique aux rayons solaires (le visage par exemple) visualise la dégénérescence induite par les ultraviolets et la formation de radicaux libres.

Cette élastose solaire touche la partie moyenne du derme ou les fibres élastiques sont irrégulières et agglutinées les unes aux autres. Les fibres en paquet disloquent les faisceaux de collagène expliquant la perte d'élasticité et la formation des rides.

### Principes actifs utilisés :

Les compositions dermo-cosmétiques sont définies à la revendication 1 de la présente invention et l'utilisation de telles compositions en cosmétologie est définie à la revendication 12 de la présente invention.

Les compositions dermo-cosmétiques renferment/comprennent à titre de principes actifs, une association synergique d'argent colloïdal et d'acide désoxyribonucléique et sont caractérisées en ce que l'argent colloïdal est plus particulièrement une solution aqueuse d'argent électro colloïdal.

Les compositions selon la présente invention peuvent contenir de la caféine.

L'argent électro colloïdal des compositions selon la présente invention peut contenir de 80 à 96 % d'ions argent et 4 à 20 % de particules d'argent.

La taille des particules d'argent peut être comprise entre 0,0008 et 0,04 microns.

La solution d'argent colloïdal peut contenir de 5 à 20 ppm d'argent.

La solution d'argent colloïdal peut contenir 10 ppm d'argent.

Les compositions selon la présente invention peuvent renfermer de 1 à 90 % d'argent colloïdal.

L'acide désoxyribonucléique des compositions selon la présente invention peut être plus particulièrement l'acide désoxyribonucléique hautement polymérisé (ADN HP).

L'ADN hautement polymérisé est plus particulièrement l'ADN HP sous forme de sel de sodium.

Les compositions selon la présente invention peuvent renfermer de 0,1 à 5 % d'ADN HP.

Les compositions selon la présente invention peuvent renfermer de 0,1 à 3 % de caféine.

Les compositions selon la présente invention peuvent être utilisées en cosmétologie dans le traitement des troubles du vieillissement et plus particulièrement pour l'activité antirides.

### L'argent colloïdal :

Il faut savoir qu'il y a quatre produits différents sur le marché appelés argent colloïdal ou colloïde d'argent.

Le premier type est le produit classique, utilisé dans nos formulations, appelé argent électro colloïdal. Ce produit est préparé par la méthode de l'arc électrique dans de l'eau désionisée, ou par la méthode de l'électrolyse à bas voltage dans de l'eau distillée. Ce produit est habituellement trouvé en concentration de 3 à 20 ppm. Il est formé de particules microscopiques d'argent pur élémentaire suspendu dans de l'eau. Chaque particule d'argent possède une charge électrique positive. Le colloïde d'argent ainsi préparé est totalement transparent.

Le deuxième est appelé colloïde d'argent aux protéines, ce produit au niveau chimique, attache des particules d'argent microscopiques à une molécule de protéine.

Le troisième, c'est le groupe des sels d'argent, par exemple le citrate d'argent.

Le quatrième est parfois appelé «poudre d'argent». Ce produit fut développé par les russes et résulte d'un fil d'argent pur qui est désintégré par une décharge électrique de haut voltage. Cette poussière microscopique est collectée et dissoute dans de l'eau.

L'argent électro colloïdal est considéré comme colloïde à cause de la taille des particules et ionique en fonction de sa charge positive.

La plupart des études biologiques démontrent que l'argent électro colloïdal possède des actions pharmacologiques plus performantes que les autres colloïdes d'argent.

Les compositions de la présente invention renferment de 1 à 90% d'argent colloïdal.

Les activités pharmacologique et biologique de l'argent électro colloïdal les plus intéressantes pour une utilisation en cosmétologie sont les suivantes :

### Activités anti-radicalaire et régénération tissulaire :

L'argent colloïdal possède une action anti-radicalaire. Le Dr BECKER en 1985, étudie le mécanisme par lequel les ions argent régénèrent les tissus. Les ions argent forment un complexe avec les cellules vivantes, pour produire des cellules souches responsables de la régénération tissulaire.

### Activité antibactérienne :

De nouveaux tests bactériologiques ont démontrés l'efficacité de l'argent colloïdal contre les micro-organismes pathogènes. Une étude de UCLA de 1988, conclue à l'action antibactérienne vis-à-vis des streptocoques pyogènes, Staphylocoques dorés.

Helen BUCKLEY (Temple University Philadelphie 1995) a utilisé avec efficacité de très petites doses d'argent colloïdal, sur plusieurs variétés de candida albicans et plusieurs cryptocoques.

Dans une étude réalisée par l'institut de microbiologie à Rome et publiée dans Microbiologie appliquée de l'environnement en décembre 1992, différentes formes d'argent furent testées pour vérifier leur capacité à tuer les microorganismes.

L'argent electro colloïdal fut plus performant que le nitrate d'argent, le chlorure d'argent et le sulfadiazine d'argent, pour sa capacité à agir comme germicide à large spectre d'activité et sur toutes les espèces de bactéries et champignons.

### Activité sur le système immunitaire :

Jason HENRY a testé l'argent colloïdal sur une levure pathogène (S.cerivisae) et rapporte qu'une seule application du produit dosé à 10 ppm, était capable de stopper le développement de la levure pendant 24 heures, ce qui permettait au système immunitaire d'avoir le temps de réagir.

### Activités anti-inflammatoire et antalgique:

Le British Medical Journal communique également que l'argent colloïdal possède une activité anti-inflammatoire et antalgique.

### Toxicologie :

L'argent colloïdal Sovereign Silver, commercialisé par la société Natural Immunogenics, dosé à 10 ppm à été testé dans un laboratoire approuvé par la FDA (Covance laboratories Inc ). Cette étude toxicologique confirme la parfaite tolérance et l'innocuité de ce produit (rapport du 20 mars 2003).

### Acide désoxyribonucléique :

L'acide désoxyribonucléique (ADN) est une molécule bien connu des biologistes depuis les travaux de WATSON et CRIK (Prix NOBEL).

L' ADN utilisé dans nos formulations est plus particulièrement l'ADN hautement polymérisé sous forme de son sel de sodium commercialisé par la société JAVENECH.

Cette macromolécule d'origine marine se présente sous la forme de longues fibres blanches. Cette apparence fibreuse est caractéristique de la super organisation en double hélice de ce bio-polymère.

L'extraction de l'ADN HP (hautement polymérisé) par des techniques non dénaturantes permet d'assurer une parfaite protection de la structure moléculaire préservant son activité physiologique.

### Les principales caractéristiques biologiques de l'ADN HP sont les suivantes :

### Action hydratante:

L'ADN HP est un excellent agent hydratant de la peau. Ainsi au niveau cellulaire, lorsqu'elles diffusent les molécules d'ADN HP se lient à un volume de solution aqueuse supérieur à 10 000 fois leur propre volume.

Les compositions de la présente invention renferment de 0,1 à 5% d'ADN HP.

### Action anti-oxydante :

Par piégeage des radicaux °OH à l'intérieur de la double hélice. La biomolécule d'ADN HP présente l'avantage par rapport à la plupart des autres substances de même activité, de ne pas générer, après captage des radicaux libres, un dérivé susceptible d'altérer d'autres constituants de proximité.

Le radical °OH est fixé sur les bases de la molécule en particulier sur la guanine, en donnant un composé stable : la 8 hydroxyguanoside.

Ses propriétés anti-lipoperoxydantes (piégeage des radicaux °OH impliqués dans l'initiation de la peroxydation lipidique) peuvent être mises à profit dans la protection de l'oxydation des lipides membranaires de la peau, ainsi que dans les crèmes, afin de protéger la phase huileuse.

L'ADN HP est également susceptible d'inhiber les élastases et plus particulièrement l'élastase des fibroblastes de la peau humaine, principalement responsable de la lyse des fibres élastiques du derme au cours du vieillissement.

### Action cicatrisante :

Cette activité est mise en évidence sur les plaies de la cornée, d'où son excellente tolérance pour la muqueuse oculaire (utilisation dans les produits « contour des yeux »)

Aux effets biologiques de l'ADN HP vient s'ajouter une caractéristique physique importante venant renforcer son rôle protecteur des structures cutanées. Sous une faible épaisseur 1mm, une solution de 1% d'ADN HP absorbe la totalité du rayonnement UV de faible intensité, compris entre 200 et 300 nm, qui sont ceux qui endommage l'ADN cellulaire.

Des expériences réalisées in vitro ont montré que l'ADN HP stimulait la synthèse des collagènes et des protéoglycanes.

### Caféine :

Ce produit est utilisé dans nos formulations, pour son action lipolytique, plus particulièrement dans les formulations «contour des yeux» qui confèrent une action anticernes performante.

Les compositions de la présente invention renferment de 0,1 à 3% de caféine.

### Association argent colloïdal et ADN HP :

Les activités synergiques de l'association argent colloïdal et de l'ADN HP selon l'invention ont été démontrées in vitro et in vivo dans les indications suivantes :

### Action anti-radicalaire (in vitro) :

La peroxydation lipidique est un cas typique de réaction en chaîne induite par voie radicalaire. L'oxydation des lipides membranaires aboutit à la formation de lipoperoxydes qui se décomposent en différents produits de fragmentation dont certains sont très toxiques et agressifs pour la peau.

L'un des produits de fragmentation le plus important et le plus agressif est un aldéhyde, le malone dialdéhyde (MDA) qui manifeste une toxicité redoutable en pontant transversalement les protéines, les lipides intracellulaires et l'ADN.

Il apparaît donc que les radicaux libres et la cascade de réaction en chaîne qu'ils provoquent au sein de l'organisme, jouent un rôle essentiel dans le processus de vieillissement cutané.

Dans le but de lutter contre cette action radicalaire, la demanderesse propose une association d'argent colloïdal et d'ADN HP.

A cet effet chacun des deux actifs a été soumis au traitement d'initiation de la peroxydation lipidique suivant :
- La peroxydation d'une émulsion d'acide linoléique a été induite par des radicaux hydroxyle °OH.
- Un effet synergique (gain de protection observé allant de 54 % à 215 % par rapport à un effet additif). Cette action synergique est plus particulièrement marquée pour une quantité de 65 à 80 ml d'argent colloïdal à 10 ppm contenant 0,3 à 0,5 % d'ADN HP.

### Régénération du collagène (culture cellulaire in vitro) :

Les UV perturbent le métabolisme cellulaire, en particulier celui des fibroblastes.

Ainsi des fibroblastes provenant d'une zone cutanée et exposés aux UV perdent leur capacité de synthèse des macromolécules et plus particulièrement du collagène, selon les travaux de OIKARINEN et col Connective tissue alteration in skin exposed to natural and therapeutic UV radiations. Photodermatology 2 p 15-26 (1985).

Dans ces conditions expérimentales, on a observé une forte potentialisation de la régénération du collagène avec l'association argent colloïdal et ADN HP.

Les résultats les plus intéressants (régénération de 65 % à 185 % par rapport à un effet additif) ont été observés pour une quantité de 60 ml à 80 ml d'argent colloïdal à 10 ppm contenant de 0,3 % à 0,5 % d'ADN HP.

### Etudes cliniques : action anti-rides

La formulation de l'exemple 1 a été testée en clinique en utilisant une méthode d'appréciation de l'activité antirides.

La méthode utilisée est la technique des empreintes cutanées associée à l'analyse macro photographique.

Les résultats de cette étude effectuée sur 78 femmes de 30 à 82 ans pendant trois mois, sont mentionnés en % de diminution des rides dans le tableau suivant :
**% de diminution des rides**

| Temps | 1 mois | 2 mois | 3 mois |
|---|---|---|---|
| Rides légères | 37 % | 64 % | 88 % |
| Rides moyennes | 21 % | 38 % | 67 % |
| Rides profondes | 15 % | 26 % | 39 % |

Les résultats avec l'ADN HP seul dosé à 500 mg dans les excipients de la crème de l'exemple 1 sont les suivants :
**% de diminution des rides**

| Temps | 1 mois | 2 mois | 3 mois |
|---|---|---|---|
| Rides légères | 9 % | 12 % | 17 % |
| Rides moyennes | 7 % | 9 % | 11 % |
| Rides profondes | 2 % | 3 % | 3 % |

Les résultats avec 70 ml d'argent colloïdal dosé à 10 ppm dans les excipients de la crème de l'exemple 1 sont les suivants :
**% de diminution des rides**

| Temps | 1 mois | 2 mois | 3 mois |
|---|---|---|---|
| Rides légères | 12 % | 16 % | 22 % |
| Rides moyennes | 9 % | 10 % | 12 % |
| Rides profondes | 3 % | 5 % | 6 % |

Ces résultats confirment la très forte potentialisation de l'action antirides de l'argent colloïdal en association avec l'ADN HP.

Compte tenu de ces résultats, la Demanderesse propose des compositions cosmétiques à base d'une association synergique d'argent colloïdal et d'ADN HP utiles dans le traitement du vieillissement cutané physiologique et actinique, éventuellement en association avec de la caféine.

L'argent colloïdal utilisé est un argent électro colloïdal contenant de 80 à 96 % d'argent ionique Ag+ et 4 à 20 % de particules. La taille des particules d'argent varie de 0,0008 microns à 0,04 microns.

La teneur en ppm (partie par million) de la solution d'argent colloïdal est comprise entre 5 ppm et 20 ppm.

Il faut noter également que les fortes concentrations de solution d'argent colloïdal permettent de diminuer de façon significative le taux des conservateurs dans nos formulations cosmétiques.

L'argent colloïdal utilisé est plus particulièrement celui commercialisé par la société Natural Immunogenics dosé à 10 ppm.

L' ADN utilisé est plus particulièrement l'ADN hautement polymérisé (HP) sous forme de sel de sodium commercialisé par la société JAVENECH.

Les formulations topiques de la nouvelle association de principes actifs selon la présente invention sont illustrées par les exemples non limitatifs suivants :

| | | |
|---|---|---|
| **Exemple 1 :** | Crème mixte émulsion fluide H/E | |
| | Principes actifs : solution à 10 ppm d'argent colloïdal 70 à 80 ml | |
| | ADN HP - 500 mg | |
| | Caféine - 500 mg | |
| | Excipients naturels d'origine végétale : | |
| | Huile d'amande douce bio : | 1 à 5 % |
| | Huile d'argan bio : | 1 à 5 % |
| | Beurre de karité bio : | 1 à 5 % |
| | Extrait huileux d'aloe vera bio : | 1 à 5% |
| | Cetearylalcool, cetearylglucoside : | 5 à 10 % |
| | Hydrogenated vegetable glycerides : | 1 à 5 % |
| | Glycérine : | 1 à 5 % |
| | Dicaprylyl carbonate : | 1 à 5 % |
| | Caprylic/caprictriglycerides : | 1 à 5 % |
| | Sodium stearoyl glutamate : | inférieur à 1% |
| | Tocopheryl acetate : | inférieur à 1 % |
| | Xanthan gum : | inférieur à 1 % |
| | Conservateurs ECOCERT : | inférieur à 1 % |
| | Parfum hypoallergénique : | inférieur à 1 % |
| | | |
| **Exemple 2 :** | Crème pour peau sèche | |
| | Principes actifs : solution à 10 ppm d'argent colloïdal 60 à 80 ml | |
| | ADN HP 900 à 1000 mg | |
| | Conservateurs ECOCERT : inférieur à 1 % | |
| | Parfum hypoallergénique : inférieur à 1 % | |
| | Autres excipients naturels d'origine végétale : QSP 100 g | |
| | | |
| **Exemple 3 :** | Crème pour peau grasse | |
| | Principes actifs : solution à 10 ppm d'argent colloïdal 65 à 85 ml | |
| | ADN HP : 800 mg | |
| | Caféine : 750 mg | |
| | Conservateurs ECOCERT : inférieurs à 1 % | |
| | Parfum hypoallergénique : inférieur à 1 % | |
| | Autres excipients naturels d'origine végétale : QSP 100 g | |
| | | |
| **Exemple 4 :** | Démaquillant visage | |
| | Principes actifs : solution à 10 ppm d'argent colloïdal 60 à 80 ml | |
| | ADN HP : 350 mg | |
| | Conservateurs ECOCERT : inférieur à 1 % | |
| | Parfum hypoallergénique : inférieur à 1 % | |
| | Autres excipients naturels d'origine végétale : QSP 100ml. | |
| | | |
| **Exemple 5 :** | Eau tonique | |
| | Principes actifs : solution à 10 ppm d'argent colloïdal 70 à 80 ml | |
| | ADN HP : 250 mg | |
| | Conservateurs ECOCERT : inférieurs à 1 % | |
| | Parfum hypoallergénique : inférieur à 1 % | |
| | Autres excipients naturels d'origine végétale : QSP 100 ml. | |
| **Exemple 6 :** | Crème contour des yeux | |
| | Principes actifs : Solution à 10 ppm d'argent colloïdal 60 à 80 ml | |
| | ADN HP : 1000 à 1200 mg | |
| | Caféine : 500 à 800 mg | |
| | Conservateurs ECOCERT : inférieurs à 1 % | |
| | Parfum : Hypoallergénique : inférieur à 1 % | |
| | Autres excipients naturels d'origine végétale : QSP 100 g. | |
| | | |
| **Exemple 7 :** | Baume à lèvres | |
| | Principes actifs : Solution à 20 ppm d'argent colloïdal 2 à 5 ml | |
| | ADN HP : 500 à 900 mg | |
| | Conservateurs ECOCERT : inférieurs à 1% | |
| | Parfum hypoallergénique : inférieur à 1 % | |
| | Autres excipients naturels d'origine végétale : QSP 100 g. | |
| | | |
| **Exemple 8 :** | Savon liquide | |
| | Principes actifs : Solution à 20 ppm d'argent colloïdal 70 à 90 ml | |
| | ADN HP : 300 à 500 mg | |
| | Caféine : 300 à 500 mg | |
| | Conservateurs ECOCERT : inférieurs à 1 % | |
| | Parfum hypoallergénique : inférieur à 1 % | |
| | Autres excipients naturels d'origine végétale : QSP 100 ml. | |
| | | |
| **Exemple 9 :** | Savon | |
| | Principes actifs : solution à 15 ppm d'argent colloïdal 1 à 5 ml | |
| | ADN HP 100 à 200 mg | |
| | Conservateurs ECOCERT : inférieurs à 1 % | |
| | Parfum hypoallergénique : inférieur à 1 % | |
| | Autres excipients naturels d'origine végétale : QSP 100 g. | |
| | | |
| **Exemple 10 :** | Crème hydratante pour les mains | |
| | Principes actifs : Solution à 10 ppm d'argent colloïdal 60 à 90 ml | |
| | ADN HP : 200 à 500 mg | |
| | Conservateurs ECOCERT : inférieurs à 1 % | |
| | Parfum hypoallergénique : inférieur à 1 % | |
| | Autres excipients naturels d'origine végétale : QSP 100 g. | |
| | | |
| **Exemple 11 :** | Bain douche | |
| | Principes actifs : Solution à 10 ppm d'argent colloïdal 70 à 90 ml | |
| | ADN HP : 100 à 200 mg | |
| | Conservateurs ECOCERT : inférieurs à 1 % | |
| | Parfum hypoallergénique : inférieur à 1 % | |
| | Autres excipients naturels d'origine végétale : QSP 100 ml | |
| | | |
| **Exemple 12 :** | Lait corporel hydratant | |
| | Principes actifs : Solution à 10 ppm d'argent colloïdal 65 à 85 ml | |
| | ADN HP : 500 à 1000 mg | |
| | Conservateurs ECOCERT : inférieurs à 1 % | |
| | Parfum hypoallergénique : inférieur à 1 % | |
| | Autres excipients naturels d'origine végétale : QSP 100ml | |
| | | |
| **Exemple 13 :** | Huile de bain | |
| | Principes actifs : solution à 10 ppm d'argent colloïdal 1 à 3 ml | |
| | ADN HP 100 à 200 mg | |
| | Conservateurs ECOCERT : inférieurs à 1 % | |
| | Parfum hypoallergénique : inférieur à 1 % | |
| | Autres excipients naturels d'origine végétale : QSP 100 ml | |
| | | |
| **Exemple 14 :** | Crème solaire | |
| | Principes actifs : Solution à 10 ppm d'argent colloïdal 65 à 80 ml | |
| | ADN HP : 1000 à 1500 mg | |
| | Conservateurs ECOCERT : inférieurs à 1 % | |
| | Parfum hypoallergénique : inférieur à 1 % | |
| | Autres excipients naturels d'origine végétale : QSP 100g | |
| | | |
| **Exemple 15 :** | Lait apaisant après solaire | |
| | Principes actifs : Solution à 15 ppm d'argent colloïdal 70 à 80 ml | |
| | ADN HP : 500 à 750 mg | |
| | Conservateurs ECOCERT : inférieurs à 1 % | |
| | Parfum hypoallergénique : inférieur à 1 % | |
| | | |
| **Exemple 16 :** | Cold cream | |
| | Principes actifs : Solution à 20 ppm d'argent colloïdal 5 à 10 ml | |
| | ADN HP : 300 à 500mg | |
| | Conservateurs ECOCERT : inférieurs à 1 % | |
| | Parfum hypoallergénique : inférieur à 1 % | |
| | Autres excipients naturels d'origine végétale : QSP 100g | |
| | | |
| **Exemple 17 :** | Crème teintée halée | |
| | Principes actifs : Solution à 10 ppm d'argent colloïdal 65 à 80 ml | |
| | ADN HP : 300 à 500 mg | |
| | Conservateurs ECOCERT : inférieurs à 1 % | |
| | Parfum hypoallergénique : inférieur à 1 % | |
| | Autres excipients naturels d'origine végétale : QSP 100g | |
| | | |
| **Exemple 18 :** | Eau de toilette | |
| | Principes actifs : Solution à 5 ppm d'argent colloïdal 30 à 50 ml | |
| | ADN HP : 100à 250mg | |
| | Parfum hypoallergénique : inférieur à 1 % | |
| | Alcool éthylique à 95° : QSP 100ml | |

## Revendications

1. Compositions dermo-cosmétiques renfermant à titre de principes actifs, une association synergique d'argent colloïdal et d'acide désoxyribonucléique **caractérisées en ce que** l'argent colloïdal est une solution aqueuse d'argent électro colloïdal, chaque particule possédant une charge électrique positive, ladite solution aqueuse d'argent électro colloïdal étant formée de particules microscopiques d'argent pur élémentaire suspendu dans de l'eau.

2. Compositions selon la revendication 1, **caractérisées en ce qu'**elles peuvent contenir de la caféine.

3. Compositions selon la revendication 1, **caractérisées en ce que** l'argent électro colloïdal contient de 80 à 96 % d'ions argent et 4 à 20 % de particules d'argent.

4. Compositions selon la revendication 3, **caractérisées en ce que** la taille des particules d'argent est comprise entre 0,0008 et 0,04 microns.

5. Compositions selon les revendications 1 à 4, **caractérisées en ce que** la solution d'argent colloïdal contient de 5 à 20 ppm d'argent.

6. Compositions selon la revendication 5, **caractérisées en ce que** la solution d'argent colloïdal contient 10 ppm d'argent.

7. Compositions selon l'une des revendications 1 à 6, **caractérisées en ce qu'**elles renferment de 1 à 90 % d'argent colloïdal.

8. Compositions selon les revendications 1 ou 2, **caractérisées en ce que** l'acide désoxyribonucléique est plus particulièrement l'acide désoxyribonucléique hautement polymérisé (ADN HP).

9. Compositions selon la revendication 8, **caractérisées, en ce que** l'ADN polymérisé est plus particulièrement l'ADN HP sous forme de sel de sodium.

10. Compositions selon l'une des revendications 1 à 9, **caractérisées en ce qu'**elles renferment de 0,1 à 5 % d'ADN HP.

11. Compositions selon l'une des revendications 1 à 10, **caractérisées en ce qu'**elles renferment de 0,1 à 3 % de caféine.

12. Utilisation non-thérapeutique en cosmétologie des compositions telles que définies à l'une quelconque des revendications 1 à 11 dans le traitement des troubles du vieillissement et plus particulièrement pour l'activité antirides.

## Patentansprüche

1. Dermokosmetische Zusammensetzungen, die als aktive Prinzipien eine synergetische Kombination aus kolloidalem Silber und Desoxyribonukleinsäure einschließen, **dadurch gekennzeichnet, dass** das kolloidale Silber eine wässrige Lösung elektrokolloidalen Silbers ist, wobei jedes Partikel eine positive elektrische Ladung besitzt, wobei die wässrige Lösung elektrokolloidalen Silbers von mikroskopischen Partikeln reinen elementaren Silbers, suspendiert in Wasser, gebildet ist.

2. Zusammensetzungen nach Anspruch 1, **dadurch gekennzeichnet, dass** sie Koffein enthalten können.

3. Zusammensetzungen nach Anspruch 1, **dadurch gekennzeichnet, dass** das elektrokolloidale Silber 80 bis 96 % Silberionen und 4 bis 20 % Silberpartikel enthält.

4. Zusammensetzungen nach Anspruch 3, **dadurch gekennzeichnet, dass** die Größe der Silberpartikel zwischen 0,0008 und 0,04 Mikron inklusive ist.

5. Zusammensetzungen nach den Ansprüchen 1 bis 4, **dadurch gekennzeichnet, dass** die Lösung kolloidalen Silbers 5 bis 20 ppm Silber enthält.

6. Zusammensetzungen nach Anspruch 5, **dadurch gekennzeichnet, dass** die Lösung kolloidalen Silbers 10 ppm Silber enthält.

7. Zusammensetzungen nach einem der Ansprüche 1 bis 6, **dadurch gekennzeichnet, dass** sie 1 bis 90 % kolloidales Silber einschließen.

8. Zusammensetzungen nach den Ansprüchen 1 oder 2, **dadurch gekennzeichnet, dass** die Desoxyribonukleinsäure insbesondere hochpolymerisierte Desoxyribonukleinsäure (DNA HP) ist.

9. Zusammensetzungen nach Anspruch 8, **dadurch gekennzeichnet, dass** die hochpolymerisierte DNA insbesondere die DNA HP in Form von Natriumsalz ist.

10. Zusammensetzungen nach einem der Ansprüche 1 bis 9, **dadurch gekennzeichnet**, sie 0,1 bis 5 % DNA HP einschließen.

11. Zusammensetzungen nach einem der Ansprüche 1 bis 10, **dadurch gekennzeichnet, dass** sie 0,1 bis 3 % Koffein einschließen.

12. Nichttherapeutische Verwendung in der Kosmetologie der Zusammensetzungen nach einem der Ansprüche 1 bis 11 bei der Behandlung von Alterserscheinungen und insbesondere für die Anti-Falten-Aktivität.

## Claims

1. Dermo-Cosmetic compositions containing as active principles a synergistic association of colloidal silver and deoxyribonucleic acid, **characterized in that** the colloidal silver is an aqueous solution of electro colloidal silver, each particle having a positive electric charge, the so-called aqueous solution of electro colloidal silver being formed of microscopic particles of pure elemental silver suspended in water.

2. Compositions according to claim 1, **characterized in that** they may contain caffeine.

3. Compositions according to claim 1, **characterized in that** the electro colloidal silver contains from 80 to 96% of silver ions and 4 to 20% of silver particles.

4. Compositions according to claim 3, **characterized in that** the size of the silver particles is between 0.0008 and 0.04 microns.

5. Compositions according to claims 1 to 4, **characterized in that** the solution of colloidal silver contains from 5 to 20 ppm of silver.

6. Compositions according to claim 5, **characterized in that** the colloidal silver solution contains 10 ppm of silver.

7. Compositions according to one of claims 1 to 6, **characterized in that** they contain 1 to 90% of colloidal silver.

8. Compositions according to claims 1 or 2, **characterized in that** the deoxyribonucleic acid is more particularly the deoxyribonucleic acid highly polymerized (HP DNA).

9. Compositions according to claim 8, **characterized, in that** the highly polymerized DNA is more particularly HP DNA in the form of sodium salt.

10. Compositions according to one of claims 1 to 9, **characterized in that** they contain 0.1 to 5% HP DNA.

11. Compositions according to one of claims 1 to 10, **characterized in that** they contain from 0.1 to 3% caffeine.

12. Non-therapeutic use in cosmetology of compositions as defined in any one of claims 1 to 11 in the treatment of disorders of ageing and more particularly for anti-wrinkle activity.
